# EUROPEAN PATENT APPLICATION

(11) **EP 0 696 451 A1**
(43) Date of publication of application: **14.02.1996**
(21) Application number: 95305329.5
(22) Date of filing: 31.07.1995
(51) Int. Cl.: A61K 7/48, A61K 9/06, A61K 9/107, A61K 31/60, A61K 31/05, A61K 31/19, A61K 47/42

(54) **Cosmetic formulations having keratolytic and/or anti-acne activity, their preparation and use**

(30) Priority: 09.08.1994 US 288098
(71) Applicant: Revlon Consumer Products Corporation, New York, NY 10022 (US)
(72) Inventor: Wolf, Barbara Ann, Scarsdale, New York 10583 (US); Snyder, Florence, Sayreville, New Jersey 08872 (US)
(74) Representative: Barrett-Major, Julie Diane

(57) **Abstract**

A cosmetic formulation with anti-acne and/or keratolytic activity comprises from about 0.01 to 25% by weight of the total formulation of a keratolytic compound complexed to a complexing molecule; and from about 75 to about 99.95% by weight of the total formulation of a cosmetically acceptable carrier therefor.

## Description

The invention relates to cosmetic compositions having keratolytic and/or anti-acne activity, their preparation and their use.

**Acne vulgaris** is reported to be the most common skin disease, affecting approximately eighty percent of the teenage population, and in some cases persisting into the third and fourth decades of life. The pathology of acne is believed first to involve the formation of comedones which are solid, horny masses of tightly-packed keratinized cells which plug skin pores and follicles. These comedone plugs are white when first formed (whiteheads), but through continued growth and deposition of melanin become blackheads. As the comedone enlarges through continued accumulation of keratinized cells, pressure builds up within the follicles and they eventually rupture, dumping the contents (consisting of horny material, sebum, and bacteria) into the skin. This provokes an inflammatory response; when the rupture is small, pustules or pimples develop; and when the follicle completely ruptures, cystic nodules result.

It is well known that **Acne vulgaris** can be treated by application of agents which dry and peel the skin to remove the keratinous plugs. Well-known keratolytic agents are sulphur, resorcinol, benzoyl peroxide, salicylic acid, and hexachlorophene. Benzoyl peroxide is an antimicrobial agent which effectively suppresses the acne bacillus **Propionibacterium acnes**, an organism which has an important causal role in acne. In addition to being an effective keratolytic agent, salicylic acid also interferes with the formation of blackheads, whiteheads, and the horny masses which clog follicles.

For some years, salicylic acid, benzoyl peroxide and resorcinol have been incorporated as the active ingredient into various anti-acne formulations in association with a cosmetically-acceptable carrier therefor. However, due to the acidic nature of these active ingredients, they often exert undesirable effects or requirements on the carrier. Salicylic acid, for example, is not water soluble; it can only solubilized in oils or alcohol. When salicylic acid is incorporated into these formulations at a pH of above 3, it converts to salicylate and causes stability problems in the formulation. Also, salicylic acid is light-sensitive above a certain pH. Make-ups made with salicylic acid are known to fade on exposure to light and can be slightly irritating in sensitive-skinned individuals who are prone to skin irritation. There is therefore a need for anti-acne formulations having improved stability and aesthetic properties.

It has most unexpectedly been discovered that if keratolytic compounds, particularly those known to also have anti-acne activity, are bound to certain complexing molecules, the resulting complex will remain stable in cosmetic formulations. When the keratolytic complex is applied to the skin in association with a cosmetically-acceptable carrier, the keratolytic compound becomes disassociated from the complexing molecule on the skin and is absorbed into the skin to provide the desired keratolytic and/or anti-acne effect. Particularly, in the case of salicylic acid, the complexation of the salicylic acid in the cosmetic formulation is believed to prevent the conversion of salicylic acid to salicylate, and therefore enhances the stability and aesthetics of the cosmetic preparation.

The present invention therefore provides a cosmetic formulation comprising:
from about 0.01 to about 25% by weight of the total formulation of a keratolytic compound complexed to a complexing molecule; and
from about 75 to about 99.99% by weight of the total formulation of a cosmetically acceptable carrier therefor.

The term "keratolytic compound" as used herein means a chemical compound having keratolytic and/or anti-acne activity.

The cosmetic formulations of the invention preferably comprise about 0.1-30% w/w of the complex and about 70-99.9% w/w of the carrier. Preferably the formulations comprise about 0.1-10% w/w complex and 90-99.9% w/w carrier.

The keratolytic compounds which are suitable for use in accordance with this invention contain an acidic group or tend to be acidic and are capable of binding with a free functional group such as the free amino group of a protein or polymer. The binding may be ionic, covalent, or through Van der Waals forces. Keratolytic compounds capable of forming ionic bonds include salicylic acid and resorcinol. Benzoyl peroxide will bond to complexing molecules through Van der Waals forces. Hexachlorphene is also a suitable keratolytic compound.

A wide variety of complexing molecules are suitable, so long as they are capable of forming a complex with the keratolytic compound, and include those which possess at least one free functional group such as amino or hydroxy.

Animal protein or peptides such as collagen, placental proteins, serum proteins, amylase, casein, urease, keratin, silk, hydrolyzed animal protein, albumin, etc. are well known in the cosmetic art and are capable of ionic bonding to the keratolytic compound. The preferred complexing molecules in this category are hydrolyzed animal protein, collagen and keratin.

Vegetable or plant proteins or polyamino saccharides containing free amino groups are also suitable, such as soya, corn, sweet corn, lupin, wheat gluten, guar, oat, extensins, hydrolyzed vegetable proteins, and the like, as well as polymers of amino sugars such as chitosan. The preferred complexing molecules in this category are hydrolyzed vegetable protein and extensins.

A wide variety of synthetic polymers have free amine groups and are also suitable as the complexing molecule. Particularly desirable are branched polyamidoamines as set forth in U.S. patent specifications nos. 4,435,548 (European patent specification no. 66 366), 4,737,550, 4,871,779, 4,857,599, 4,713,975 (European patent specification no. 247 629), 4,694,064 (European patent specification no. 234 408), 4,690,985, 4,631,337, 4,599,400 (European patent specification no. 195 126), 4,587,329, 4,568,737, 4,558,120 and 4,507,466 (European patent specification no. 115 771) which are hereby incorporated by reference in their entirety. These patent specifications disclose and claim dense star-type polymers with dendritic branches which have highly reactive substitutent groups.

Other synthetic polymers such as polyamides, polyanilines, polyureas, polyurethanes contain at least one free amino group and are suitable complexing molecules.

Certain other polymers which have free hydroxyl groups, such as polyvinyl alcohols, poly(2-hydroxyethyl methacrylate)(2-HEMA) and poly(2-hydroxypropyl methacrylate) (2-HPMA) will bind by Van der Waals forces to the keratolytic compound and hence are also suitable as complexing molecules.

The keratolytic compound is bonded to the complexing molecule by standard techniques known in the art. A variety of chemical reactions may be employed, depending on the complexing molecule and the keratolytic compound used. The term "complex" refers to the keratolytic compound complexed with (or bound to) the complexing molecule.

For example, salicylic acid and resorcinol have free acid groups which will ionically bond to the free amino groups of proteins or polymers. Binding of the salicylic acid to vegetable or animal proteins is achieved by simply combining approximately equal parts of both ingredients in a suitable vessel. The salicylic acid will react with the free amino groups on the complexing molecule to form a complex. Sometimes moderate heating, or the addition of a small amount of acid such as hydrochloric acid, will hasten the reaction. Salicylic acid may also be complexed to complexing molecules such as hydrolyzed vegetable proteins as described in French patent specification number 2,667,072 which is hereby incorporated by reference. In particular, a concentrated solution of salicylic acid in alcohol is mixed with a concentrated solution of hydrolyzed vegetable protein in water. The resulting dispersion is mixed under shear or by ultrasonic agitation. Depending on the type of protein selected, the complex may precipitate upon forming or may be recovered by vacuum distillation or lyophilization by a method known to those skilled in the art. The resulting powder effectively solubilizes the complexed salicylic acid which can be recovered and analyzed as salicylic acid by traditional wet chemistry. Alternatively, the salicylic acid can be rendered more water soluble by using an alkali metal salt such as sodium or potassium and complexing at a pH of 5.5 to 6.5. The powdered hydrolyzed protein is then added to a dispersion of the salicylate in a buffered water solution.

Alternatively, salicylic acid can be complexed with the dendritic polymers disclosed herein. Dendritic polymers are constructed to vary in size and shape depending upon the initiator and the molecule used to grow the shell. Of particular interest are the starburst dendrimers of the polyamidoamine (PAMAM) type because the outer shell is covered with amino groups which will complex with salicylic acid. The starting point can be either a water dispersion of dendrimer to which is added salicylic acid in a volatile solvent, or a solution of salicylate at pH 5.5 to 6.5 to which is added crystals of dendrimers. The size of the dendrimer will allow multiple sites for complexation, but not so large that solubility in water is hindered. Dendrimers having 1-10 generations are ideal. Since the dendrimers can develop holes or irregularities, some salicylic acid may be sequestered within the shell as well as complexed to the surface. As with the protein, a high complexation ratio is desirable.

Salicylic acid will also bind to synthetic polymers which have free amino groups in a manner similar to their binding to proteins. Such polymers include: polyamides -(R-CO-NH)ₙ-; polyanilines -(C₆H₆NH)ₙ-; polyureas (-R-NHCONH)ₙ; and polyurethanes -(CO-OR-OCO-NHR-R'NH)ₙ; where the branched or terminal groups have free amino groups and the interior amine groups are also available for complexing.

Polymers having free hydroxy groups will bind to salicylic acid by Van der Waals forces. Such polymers include polyvinyl vinyl alcohols -(CHₙCHOH)-; poly(2-HEMA) (-CH2-C(CH₃)(COOCH₂CH(OH)-)ₙ and poly(2-HPMA) (-CH₂-C(CH₃)(COOCH₂CH₂OH-)ₙ and (-CH₂-(CH₃)(COOCH₂CH(OH) CH₃)ₙ. Such bonds are weaker, but may be enhanced by shared electrons across the aromatic ring of salicylic acid.

Benzoyl peroxide will only form Van der Waals bonds with proteins or polymers. The complexation of benzoyl peroxide to proteins is achieved by sharing electrons across the peroxide moiety and the amido moiety in the polymer.

It is generally preferred that the complex be comprised of 40-50% by weight of the complex of the keratolytic compound and 50-60% by weight of the complex of the complexing molecule. Ratios may vary depending on the effect desired. For example, in some cases it may be desired to have small levels of the keratolytic compound deposited on the skin, in which case more appropriate ratios might range from 10-40% w/w keratolytic compound and 60-90% w/w complexing molecule, or vice versa.

It may be desired to encapsulate the complex to provide additional properties such as timed release (slow or sustained release, for example). Standard encapsulation methods are suitable, such as the methods disclosed in U.S. patent specification no. 5,194,262 (European patent specification no. 589 883) which is hereby incorporated by reference in its entirety.

The term "cosmetically acceptable carrier" generally refers to a composition applied externally to the skin or hair of the human body for the purpose of, for example, cleansing, beautifying, conditioning or protecting the body surface. A wide variety of carriers are suitable including but not limited to water-in-oil or oil-in-water emulsions in cream or lotion form, such as sunscreens, toners, astringents, facial make-ups (foundations), pressed or loose powder, concealers or other anhydrous make-ups, skin cleansing compositions, and the like. In some cases it may be desired to apply the complex to the scalp to achieve keratolytic effects, so suitable carriers also include shampoos and conditioners. All % weights referred to in the following general description of the carrier are by weight of the total formulation, unless otherwise specifically stated.

Creams generally contain about 10-90% water and 10-90% oil. The oil may be selected from low and high viscosity surface oils, volatile silicones, nonvolatile silicones, and amine functional silicones. Creams may also contain excipient(s) selected from humectants, emollients, surfactants, emulsifiers, preservatives and fragrances. For example, about 5-10% w/w humectant, 5-20% w/w emollient, and about 0.5-10% w/w surfactant or emulsifier is preferred.

Lotions are generally comprised of 20-80% w/w oil and 10-80% w/w water in emulsion form. Lotions may also contain excipient(s) selected from those mentioned above for creams. For example, about 5-10% w/w humectant, about 5-20% w/w emollient, and about 0.5-10% w/w surfactant is preferred.

Make-ups or foundations generally comprise about 5-70% w/w oil, 10-95% w/w water and about 0.01-40% w/w pigment. In addition, the makeup may contain surfactants and/or silicones as part of the oil phase; and other excipient(s) hereinbefore mentioned. Generally 0.1-10% w/w surfactant, 0.1-50% w/w silicone, 0.1-20% w/w humectant, 0.1-30% w/w emollient, and 0.1-5% w/w preservative is preferred. More preferably, the carrier is a make-up comprising 0.01-50% w/w silicone, 30-60% w/w water, 5-40% w/w pigment and 5-40% w/w oil. Especially preferred is when the composition further comprises 0.1-20% w/w humectant, 0.1-10% w/w surfactant and 0.1-5% w/w preservative.

Foundations or make-ups according to the invention have improved properties over the anti-acne make-ups currently available in that they do not change colour or fade. Normally anti-acne actives, salicylic acid in particular, react with the iron oxide pigments commonly used in make-up. However, because the keratolytic compound is bound to a complexing molecule, such reaction does not occur in the make-up composition of the invention.

Generally, blushers contain about 5-75% w/w pigment, 1-50% w/w oil, and 1-20% w/w wax. They may additionally contain one or more of 10-60% w/w water, 0.5-30% w/w surfactant, 1-10% w/w humectants, 0.1-5% w/w preservative, and 0.1-20% w/w silicone. Face powders comprise 5-75% w/w powder, 0.1-20% w/w wax, and 0.01-20% w/w pigments. They may also contain one or more of 10-60% w/w water, 0.5-30% w/w surfactant, 1-10% w/w humectants, 0.1-5% w/w preservative, and 0.1-20% w/w silicone. Anhydrous make-ups and concealers generally comprise one or more of 0.1-10% w/w surfactant, 0.1-50% w/w silicone, 0-20% w/w humectant, 0.1-30% w/w emollient, 0.1-50% w/w pigment and 0.1-5% w/w preservative.

Suitable shampoo compositions contain 1-40% w/w of a cleansing surfactant and 10-90% w/w water. Preferably the surfactants are anionic or amphoteric. The shampoo may also contain other excipients selected from surfactants, colourants, preservatives, fragrances, emulsifiers, viscosity adjusters and conditioning agents. If present, suitable ranges are 0.01-30% w/w surfactant, 0.001-5% w/w colourant, 0.0001-5% w/w preservative, 0.01-15% w/w emulsifiers, 0.01-10% w/w viscosity adjusters and 0.1-20% w/w conditioning agents.

Suitable hair conditioning formulations include 10-95% w/w water, 0.5-30% w/w conditioning ingredients such as quaternary ammonium compounds or amphoteric polymers, proteins and the like, and 1-40% w/w surfactants. Hair conditioners may also contain volatile or non-volatile silicones; preferably in an amount of about 0.5-15% w/w.

The carrier may also comprise pharmaceutical type vehicles such as ointments, gels or solutions. Suitable ointments are hydrophilic ointments (USP) or petrolatum. Solutions are made by mixing the keratolytic/complexing molecule complex in deionized water. Gels generally comprised about 1-90% w/w water and about 1-90% w/w of a suitable polymer such as polypropylene.

Suitable emollients include glyceryl stearate, cetyl alcohol, stearyl alcohol, stearyl stearate, isopropyl stearate, stearic acid, isobutyl palmitate, isocetyl stearate, oleyl alcohol, sebacates, myristates, palmitates, squalenes, glyceryl monooleate, oleic acids, lanolin, acetylated lanolin alcohols, petrolatum, mineral oils, palmitic acids, isostearyl neopentanoate, and the like.

Suitable humectants include glycerin, butylene glycol, propylene glycol, glucose, fructose, glucuronic acid, glucamine, glutamic acid, glycereth-7, glycereth-12, glycereth-26, histidine, honey, lactose, mannitol, methyl gluceth, sodium PCA (as defined in CFTA Cosmetic Ingredient Handbook page 274), PEG-10 propylene glycol, urea, xylitol, TEA-lactate, TEA-PCA, sucrose, sorbitol, PCA, sodium lactate, or mixtures thereof, and the like.

A variety of surfactants may be used in the carrier including amphoteric, anionic, cationic or nonionic surfactants. Suitable amphoteric surfactants include imidazolines, betaines and amino acid salts, and the like. Suitable anionic surfactants include fatty acid soaps, salts of higher alkyl sulphates, n-acyl sarcosinates, salts of phosphates, sulphosuccinate salts, alkyl benzene sulphonates, salts of N-acyl glutamate, polyoxethylene alkyl ether carboxylic acids, and so on. Cationic surfactants include alkyl trimethyl ammonium salts, alkyl pyridinium salts, alkyl quaternary ammonium salts, polyamine fatty acid derivatives etc. Nonionic surfactants include lipophilics such as sorbitan fatty acid esters, glycerol fatty acids, propylene glycol fatty acid esters, hydrophilics such as polyoxyethylene sorbitan fatty acid esters, polyoxyethylene glycerol fatty acid esters, polyoxyethylene fatty acid esters, polyoxyethylene alkyl ethers, pluronics, polyoxyethylene alkyl phenyl ethers, polyoxyethylene propylene glycol fatty acid esters, and so on.

Suitable pigments include organic and inorganic pigments such as talc, mica, titanium dioxide, titanated mica, iron oxides, ultramarines, chromium oxides, carmine, D&C and FD&C colours and lakes, ferric and ferrous oxides, and the like.

Suitable preservatives include the ureas such as imidazolidinyl urea, diazolidinyl urea, the parabens, quaternium 15, benzyl alcohol, phenoxyethanol and the like.

Suitable waxes include beeswax, cetyl esters, carnauba, ceresin, microcrystalline, lanoline, paraffin, ozokerite, lanolin alcohol, acetylated lanolin, candelilla, cetyl alcohol, cocoa butter, petrolatum, hydrogenated castor oil, spermacetic, bran wax, capok wax, bayberry, hydrogenated jojoba oil, hydrogenated jojoba wax, hydrogenated rice bran wax, japan wax, jojoba butter, jojoba oil, mink, montan acid, montan, ouricury, shelac, etc.

Suitable silicones include cyclomethicone, dimethicone, stearoyl dimethicone, phenyl trimethicone, dimethiconol, dimethicone copolyols, and the like.

Further suitable excipients are as set forth in the CTFA Cosmetic Ingredient Handbook, First Edition (1988), especially on pages 79-81, 75, 87-90, 90-94, 97, 33 and 63, which is hereby incorporated by reference in its entirety.

In the preferred embodiment of the invention the anti-acne active/carrier molecule complex comprises 40-50% w/w by weight plant protein (hydrolyzed vegetable protein) and 45-55% w/w salicylic acid. The preferred diluent is a make-up or skin care product.

This invention also provides a method for preparing such a formulation comprising:
a) complexing a keratolytic compound with a complexing molecule; and
b) bringing said complex produced by step (a) into association with a cosmetically acceptable carrier.

There is further provided a formulation as hereinbefore described for use as a medicament; in particular, for use as a keratolytic and/or for the treatment or prevention of acne.

The present invention also provides a complex comprising a keratolytic compound complexed to a complexing molecule, as hereinbefore described; its preparation as hereinbefore described; and its use in the manufacture of a cosmetic or medicament, as hereinbefore described.

### Examples 1 to 4: Preparation of Complexes

### Example 1

A concentrated solution of hydrolyzed vegetable protein in water is mixed with a solution of 50% salicylic acid in ethanol. The resulting dispersion is mixed under shear. The mixture is allowed to stand for approximately 24 hours. Lyophilization is performed to yield a powdered material which comprises salicylic acid complexed to the hydrolyzed vegetable protein.

### Example 2

A water dispersion of about 50wt% polyamidoamine methacrylate crystals in water is prepared. A solution of 50% salicylic acid in ethanol is slowly added to the crystals with stirring for a half hour. The mixture is vacuum distilled to yield crystals having salicylic acid complexed thereto.

### Example 3

A concentrated dispersion of polyacrylamide in water is mixed with a 50% solution of salicylic acid in ethanol with high shear mixing. After 24 hours the resulting dispersion is lyophilized to yield crystals having salicylic acid complexed thereto.

### Example 4

A 50% solution of benzoyl peroxide in ethanol is mixed with a 25% solution of hydrolyzed vegetable protein. The mixture is allowed to stand for 24 hours. The mixture is then lyophilized to obtain a powder. The powder comprises hydrolyzed vegetable protein having benzoyl peroxide complexed thereto.

### Examples 5 to 13: Formulations

The salicylic complex of Example 1 was used to prepare formulations as follows:

### Example 5

A make-up was prepared, comprising:

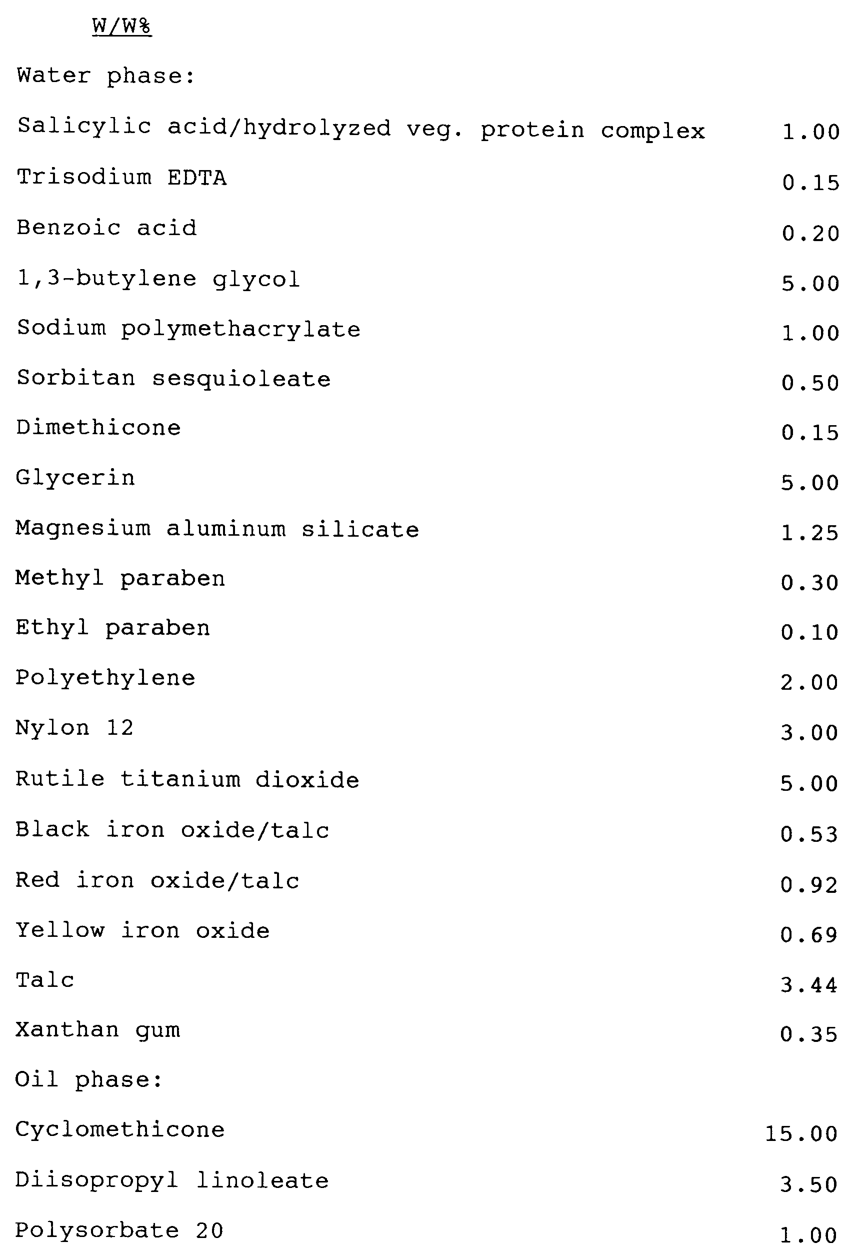

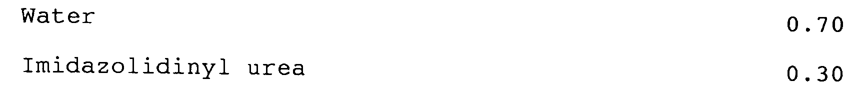

Water was heated to 60-65°C. All water phase ingredients except the urea were mixed in a colloid mill until all ingredients were dispersed. The mixture was then heated in a steam bath to 68-70°C using a sweep mixer. Next combined were the oil phase ingredients which were heated in the steam bath. When the water and oil phases were at the same temperature, they were mixed with sweep action to form the emulsion. Sweep mixing was continued at the maintenance temperature for 15-20 minutes. The mixture was then removed from the bath and allowed to cool while maintaining mixing. The urea was added when the batch temperature reach 40-45°C.

### Example 6

An oil-in-water moisturising lotion is made as follows:

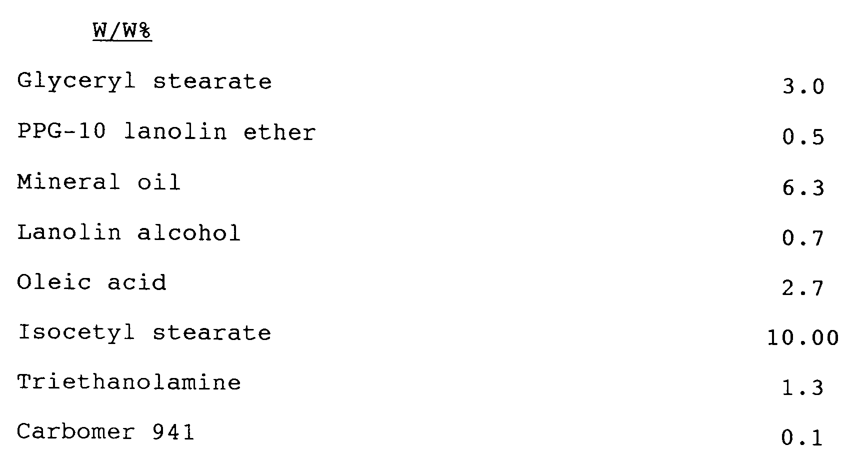

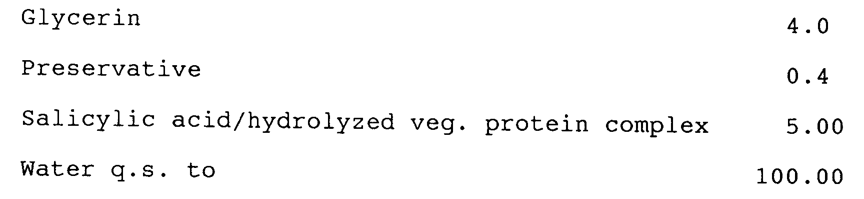

The lotion was made by mixing into about 30 grams of water the remaining ingredients.

The composition was emulsified and water added to 100 grams.

### Example 7

An oil-in-water moisturising cream is made as follows:

| W/W% | |
|---|---|
| Glyceryl stearate | 5.0 |
| Cetyl alcohol | 2.0 |
| Stearyl alcohol | 2.0 |
| Isopropyl stearate | 4.0 |
| Mineral oil | 12.0 |
| Polysorbate 60 | 1.0 |
| Glycerin | 8.0 |
| Xanthan gum | 0.25 |
| Preservative | 0.60 |
| Salicylic acid/hydrolyzed veg. protein complex | 5.00 |
| Water q.s. to | 100.00 |

The ingredients were combined and mixed to emulsify.

### Example 8

An anhydrous make-up with salicylic acid was made as follows:

| W/W% | |
|---|---|
| Stearoyl dimethicone | 12.00 |
| Phenyl trimethicone | 16.00 |
| Octyldodecanol | 12.00 |
| Dimethicone | 4.00 |
| BHA (Butylated hydroxy anisole, antioxidant) | 0.10 |
| Phenoxyethanol | 0.70 |
| Titanium dioxide | 15.00 |
| Black iron oxide/talc | 0.26 |
| Red iron oxide/talc | 2.00 |
| Yellow iron oxide/talc | 5.00 |
| Talc | 6.94 |
| Lecithin treated mica | 6.00 |
| Hydrogenated cocoa glycerides | 10.00 |
| Ceresin wax | 1.00 |
| Tribehenin | 8.00 |
| Salicylic acid/hydrolyzed veg. protein complex | 1.00 |

All ingredients except the complexed salicylic acid/protein was mixed in a steam bath until the waxes were molten. The entire batch was roller milled until the pigments were dispersed. The mixture was then returned to the steam bath and the salicylic acid complex was added. The batch was prop mixed until cooled to room temperature.

### Example 9

An anhydrous concealer stick was made as follows:

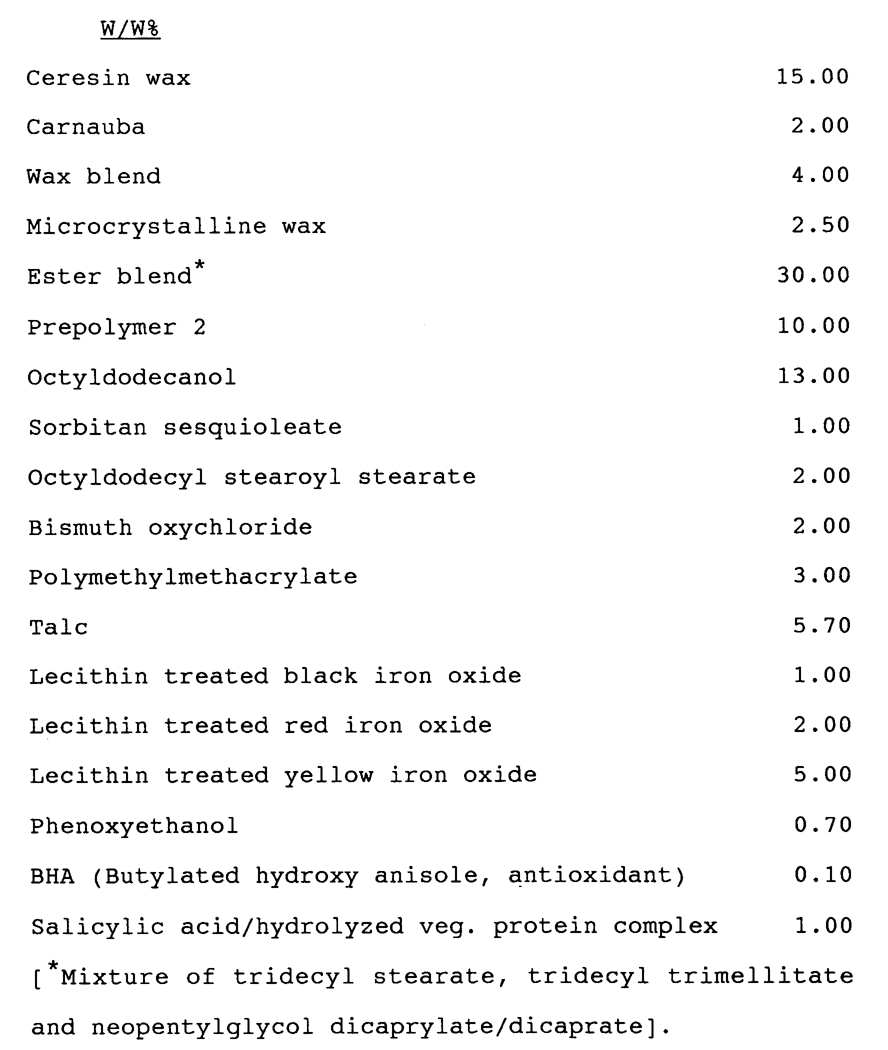

All ingredients except the salicylic acid/protein complex were mixed in a steam bath until the waxes were molten. The entire batch was roller milled until the pigments were dispersed. The batch was returned to the steam bath and the salicylic acid complex was added. The batch was prop mixed until it cooled to room temperature.
Examples 10 to 13 were prepared according to the method of Example 7:

### Example 10

A shampoo composition was made as follows:

| W/W% | |
|---|---|
| Ammonium lauryl sulphate | 10.00 |
| Cocamide diethanolamine | 4.00 |
| Cocamidopropyl betaine | 4.00 |
| Ammonium chloride | 0.80 |
| Citric acid | 0.10 |
| Salicyclic acid/ydrolyzed veg. protein complex | 5.00 |
| Water q.s. to | 100.00 |

### Example 11

A creme rinse hair conditioner was made as follows:

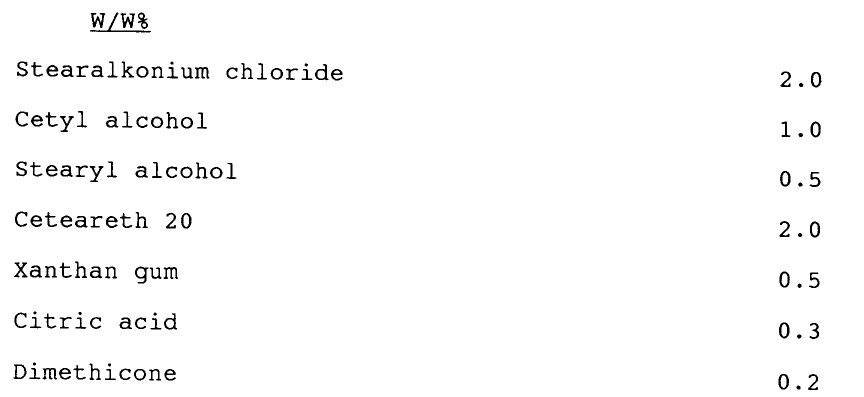

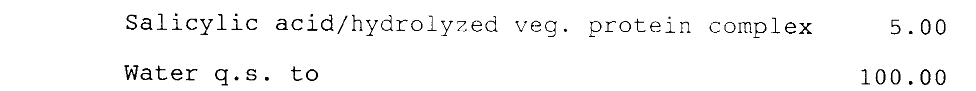

### Example 12

A toner composition was made as follows:

| W/W% | |
|---|---|
| Polysorbate 20 | 1.0 |
| Ethyl alcohol | 50.00 |
| Perfume | 8.00 |
| Salicylic acid/hydrolyzed veg. protein complex | 10.00 |
| Water q.s. to | 100.00 |

### Example 13

A cleansing cream was made as follows:

| W/W% | |
|---|---|
| Mineral oil | 20.00 |
| Beeswax | 2.00 |
| Polysorbate 40 | 8.00 |
| PEG 20 sorbitan beeswax | 2.00 |
| Stearic acid | 10.00 |
| Petrolatum | 4.00 |
| Sorbitol | 5.00 |
| Perfume | 1.00 |
| Preservative | 0.50 |
| Salicylic acid/hydroyzed veg. protein complex | 10.00 |
| Water q.s. to | 100.00 |

## Claims

1. A cosmetic formulation comprising from about 0.01 to about 25% by weight of the total formulation of a keratolytic compound complexed to a complexing molecule; and from about 75 to about 99.99% by weight of the total formulation of a cosmetically acceptable carrier.

2. A formulation according to claim 1 wherein the keratolytic compound is resorcinol, benzoyl peroxide, salicylic acid, or a mixture thereof.

3. A formulation according to claim 1 or claim 2 wherein the complexing molecule is selected from those having at least one free amino or hydroxy functional group.

4. A formulation according to any preceding claim wherein the complexing molecule is a hydrolyzed vegetable protein, a hydrolyzed animal protein, a branched polyamidoamine, a polyamide, a polyaniline, a polyurea, a polyurethane, polyvinyl alcohol derivative, or a mixture thereof.

5. A formulation according to any preceding claim wherein the cosmetically-acceptable carrier is in the form of a cream, lotion, make-up or foundation, blusher, skin cleanser, sunscreen, toner, astringent, powder, shampoo or conditioner.

6. A formulation according to any preceding claim wherein the formulation comprises 10-40% by weight of the complex of the keratolytic compound and 60-90% by weight of the complex of the complexing molecule.

7. A formulation according to any preceding claim comprising 0.01-20% by weight of the total formulation of the complex and 90-99.9% by weight of the total formulation of the carrier.

8. A formulation according to any preceding claim wherein the keratolytic compound is salicylic acid.

9. A formulation according to any preceding claim wherein the complexing molecule is a hydrolyzed vegetable protein.

10. A formulation according to any preceding claim wherein the carrier is in the form of a make-up or foundation.

11. A formulation according to any preceding claim wherein the carrier comprises 5-70% w/w oil, 10-95% w/w water, and 0.01-40% w/w pigment.

12. A formulation according to any preceding claim wherein the carrier comprises 5-40% w/w oil, 30-60% w/w water, 5-40% w/w pigment and 0.01-50% w/w silicone.

13. A formulation according to any preceding claim for use as an anti-keratolytic and/or anti-acne comprising:
0.01-50% w/w silicone
0.01-10% w/w of salicylic acid complexed with hydrolyzed vegetable protein,
0.01-40% w/w pigment, and
5-40% w/w oil.

14. A formulation according to any preceding claim further comprising 0.1-10% w/w surfactant, 0.1-20% w/w humectant, 0.1-30% w/w emollient, or mixtures thereof.

15. A formulation according to any of claims 12 to 14 wherein the silicone is a mixture of volatile and non-volatile silicones.

16. A formulation according to any preceding claim for use as a medicament.

17. A formulation according to any preceding claim for use as a keratolytic and/or for treating **Acne vulgaris**.

18. A method for preparing a formulation according to any preceding claim comprising:
a) complexing a keratolytic compound with a complexing molecule; and
b) bringing said complex produced by step (a) into association with a cosmetically acceptable carrier.

19. A method of cosmetically making-up skin which method comprises applying to the skin a cosmetic formulation according to any of claims 1 to 15.

20. A pack comprising a formulation according to any of claims 1 to 15 in a container therefor and instructions for the use thereof according to any of claims 16, 17 or 19.
